(19) Europäisches Patentamt
European Patent Office
Office européen des brevets

(11) Numéro de publication: **0 127 501**
**B1**

(12) # FASCICULE DE BREVET EUROPEEN

(45) Date de publication du fascicule du brevet: **01.04.87**

(51) Int. Cl.⁴: **C 07 C 45/50**, C 07 C 47/02

(21) Numéro de dépôt: **84400822.7**

(22) Date de dépôt: **24.04.84**

(54) **Système catalytique pour l'hydroformylation des oléfines. Procédé d'hydroformylation.**

(30) Priorité: **22.04.83 FR 8306649**

(43) Date de publication de la demande: **05.12.84 Bulletin 84/49**

(45) Mention de la délivrance du brevet: **01.04.87 Bulletin 87/14**

(84) Etats contractants désignés: **AT BE DE FR GB IT LU NL**

(56) Documents cités:
**FR - A - 2 395 246**
**GB - A - 2 055 053**
**US - A - 3 284 510**

**Le dossier contient des informations techniques présentées postérieurement au dépôt de la demande et ne figurant pas dans le présent fascicule.**

(73) Titulaire: **CDF CHIMIE SPECIALITES, Tour Aurore Place des Reflets, F-92080 La Defense Cedex 5 (FR)**

(72) Inventeur: **Bourgeois, Claude, 3 square de Toscane, F-78190 Montigny-le-Bretonneux (FR)**
Inventeur: **Demay, Claude, 5 rue Clément Ader, F-78180 Voisins-le-Bretonneux (FR)**

(74) Mandataire: **Rieux, Michel, C d F Chimie S.A. Service Propriété Industrielle Tour Aurore Place des Reflets Cédex no. 5, F-92080 Paris la Défense 2 (FR)**

**EP 0 127 501 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un système catalytique pour l'hydroformylation des oléfines encore appelée réaction OXO. Elle concerne également le procédé d'hydroformylation à partir du système catalytique.

Les procédés d'hydroformylation d'une oléfine pour préparer des aldéhydes ou des alcools ayant un atome de carbone de plus que l'oléfine initiale consistent à faire réagir cette oléfine avec un gaz de synthèse en présence d'un catalyseur complexe contenant un métal choisi dans la série des métaux de transition. Parmi ceux-ci, les métaux du groupe VIII de la cassification périodique c'est-à-dire fer, cobalt, nickel, ruthénium, rhodium, palladium, osmium, iridium, platine, sont particulièrement utilisés.

Ces métaux peuvent être employés sous forme de métaux carbonyles, mais il est reconnu que les combinaisons complexes contenant, outre le métal et l'oxyde de carbone, au moins un ligand biphyllique tel qu'une phosphine, conduisent à des produits plus linéaires et permettent de travailler à des pressions de gaz de synthèse faibles. De telles combinaisons sont, par exemple, décrites dans le brevet français no 1300404 ou encore dans le brevet des Etats-Unis d'Amérique no 3239566.

Ces métaux peuvent être utilisés isolément, mais certains brevets décrivent l'utilisation de leurs combinaisons. Ainsi dans le brevet français no 2395246 des couples de métaux associés à des phosphines sont utilisés pour hydroformyler des oléfines internes. Dans le brevet français no 2459683, le couple rhodium-cobalt associé à de la triphénylphosphine présente des avantages en ce qui concerne le viellissement du catalyseur pour l'hydroformylation du propène.

Selon l'invention le système catalytique constitué d'une combinaison complexe (1) de rhodium, d'oxyde de carbone et de ligand triorganophosphoré, d'un composé (2) de cobalt et d'un composé triorganophosphoré (3), et caractérisé en ce qu'il contient au moins un composé (4) qui est un diène conjugué ou un composé générant in situ en cours de réaction d'hydroformylation un diène conjugué, ledit diène conjugué étant choisi dans les molécules aliphatiques, alicycliques, hétérocycliques et aromatiques possédant dans leur structure le motif

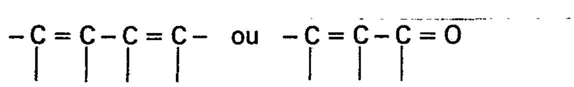

et en ce que le diène conjugué ne se trouve pas en combinaison complexe avec le rhodium ou le cobalt, présente, de manière inattendue, une activité bien supérieure à celles qui sont communément obtenues avec des catalyseurs connus. Ceci est d'autant plus surprenant que les diènes conjugués sont connus pour être des inhibiteurs, voire des poisons de catalyseurs dans la synthèse OXO: J. Falbe «New Synthesis with carbon monoxide», Springer Verlag, N.Y. 1980. On peut relever les mêmes critiques dans le brevet français no 2361324.

Par diènes conjugués sont désignées des molécules ayant dans leurs structures le motif

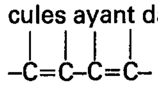

ou encore le motif

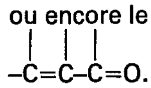

Ces motifs peuvent appartenir à des molécules aliphatiques, alicycliques, hétérocycliques ou aromatiques. Sont également comprises dans l'invention les composés dont les molécules peuvent générer in situ en cours de réaction d'hydroformylation une structure telle que celles qui sont décrites ci-dessus; à titre d'exemple, il en est ainsi des β dicétones ou des quinones qui par le jeu d'équilibres tautomères ou chimiques génèrent des diènes conjugués.

A titre d'illustrations non limitatives sont donnés quelques exemples de diènes conjugués.

– Ethyl-2 héxèn-2 al

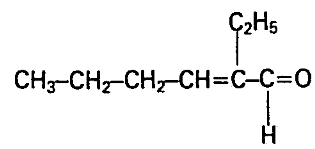

– Ethyl-2 méthyl-4 pentèn-2 al

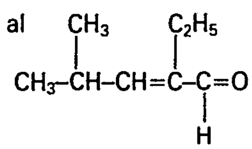

– Acétylacétone

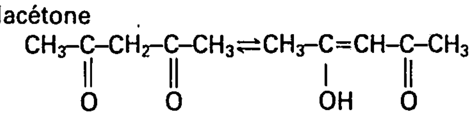

– Diphényl-1,4 butadiène-1,3

0–CH=CH–CH=CH–0

– Oxyde de mésityle

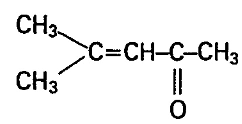

– Isophorone

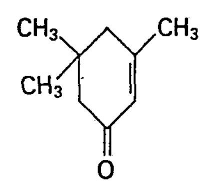

– Triphényl-1,2,5 phosphole

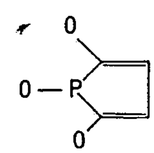

– Butadiène-1,3 $CH_2 = CH - CH = CH_2$

– Cyclopentadiène

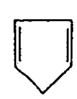

– Hydroquinone

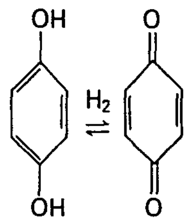

– Diméthyl-1,5,2 pyrrole carbonitrile

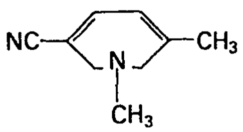

Le composé du rhodium est une combinaison complexe de rhodium, d'oxyde de carbone et de ligand triorganophosphoré. L'utilisation de telles combinaisons prises isolément comme catalyseur OXO est connue. En général les résultats les plus satisfaisants sont obtenus lorsque le ligand triorganophosphoré est choisi parmi les arylphosphines ou les arylphosphites. Un exemple d'une telle combinaison, lorsque le ligand est la triphénylphosphine, est le rhodium (I) hydrogénocarbonyl tris (triphénylphosphine) de formule H Rh CO $(P0_3)_3$.

Le cobalt peut être efficacement introduit dans le milieu de réaction sous des formes extrêmement variées. La plupart des combinaisons contenant du cobalt peuvent être utilisées. On peut par exemple introduire le cobalt sous une forme carbonylée telle que $Co_2(CO)_8$ ou H Co $(CO)_4$; on peut encore utiliser le cobalt sous la forme d'un sel minéral ou d'un sel organique tel que l'acétate de cobalt, l'acétyl-acétonate de cobalt, le benzoate ou le naphténate de cobalt. Bien évidemment le composé de cobalt utilisé ne doit pas comporter de groupement susceptible d'être un poison pour la réaction OXO elle-même; il est connu que les ions chlorures ou sulfures sont des poisons pour la synthèse OXO. De tels composés sont cités en particulier dans le livre de J. Falbe «Carbon monoxide in Organic synthesis» Springer-Verlag, N.Y. 1970 ou encore dans le brevet français no 2377991. D'une manière générale, lorsque le cobalt est introduit sous forme carbonylée, l'obtention de l'activité catalytique est quasiment instantanée. Par contre, quand le cobalt est introduit sous forme d'un sel organique ou minéral, on peut observer une période d'introduction de quelques dizaines de minutes au cours de laquelle se forme probablement in situ l'espèce catalytique active. On peut penser que le cobalt intervient en combinaison avec les divers éléments présents dans le milieu réactionnel. Cette remarque n'est qu'une hypothèse d'explication au phénomène envisagé et ne saurait en aucun cas constituer une limite au cadre de la présente invention pour laquelle d'autres explications restent bien entendu possibles.

Le composé triorganophosphoré est choisi parmi les arylphosphines ou les arylphosphites; il n'est pas obligatoirement de même nature que le ligand organophosphoré introduit avec le rhodium mais lorsque cela est le cas, il peut alors être considéré comme une excès de ce ligand dans le milieu réactionnel.

Les concentrations en rhodium peuvent être comprises entre $10^{-4}$ et $10^{-1}$ mole/litre de solution réactionnelle, mais on obtient des résultats satisfaisants pour des concentrations comprises entre $10^{-3}$ et $10^{-2}$ mole/litre.

Les quantités de cobalt peuvent être introduites à des concentrations comprises entre $10^{-4}$ et 1 mole/litre de solution réactionnelle. Lors de l'utilisation de concentrations élevées en cobalt, on peut observer dans certains cas des précipités dus à la solubilité limitée des complexes organo-métalliques dans le milieu réactionnel. Cette solubilité dépend évidemment du milieu solvant. Si on veut éviter de tels précipités, il sera nécessaire de déterminer les concentrations en cobalt permettant de ne pas dépasser les limites de solubilité. Dans la pratique on opérera généralement à des concentrations comprises entre $2\times10^{-4}$ et 0,2 mole/litre.

La concentration en diène est comprise entre $5\times10^{-3}$ et 350 g/litre de solution réactionnelle. De bons résultats sont généralement obtenus pour des concentrations comprises entre $5\times10^{-2}$ et 200 g/litre.

Il a cependant été observé que pour certains diènes, le système catalytique donne des résultats satisfaisants pour des concentrations en diène faibles, alors que des concentrations plus élevées peuvent conduire à un ralentissement de la réaction, voire à son blocage complet. Ce phénomène a par exemple été noté dans le cas de l'acroléine qui conduit à des résultats satisfaisants pour des concentrations égales à 0,4 g/litre, mais où le blocage de la réaction est constaté pour des concentrations égales à 35 g/litre. Par contre, par exemple dans le cas de l'éthyl-2 héxèn-2 al, les concentrations peuvent aller sans aucun problème jusqu'à plus de 200 g/l. On peut supposer que les propriétés du diène telles que par exemple l'encombrement stérique ou les facteurs électroniques influent sur la stabilité des complexes engagés dans le processus catalytique et que par conséquent les concentrations en diène pour lesquelles le système catalytique est particulièrement efficace pourraient varier d'un diène à l'autre. Les domaines de concentration au diène cités précédemment sont donnés à titre indicatif et conviennent en général pour la majorité des diènes; le spécialiste pourra après quelques essais classiques sélectionner dans ce domaine les valeurs les mieux appropriées, c'est-à-dire les valeurs ne conduisant pas à un ralentissement de la réaction.

Les ligands organophosphorés tels que les arylphosphines ou arylphosphites peuvent être utilisés dans des rapports molaires phosphore/rhodium supérieurs à 10.

L'hydroformylation des oléfines avec le présent système catalytique s'effectue en général à une température comprise entre, environ 60°C et environ 150°C, des températures comprises entre 80°C et 125°C étant le plus souvent utilisées, la pression totale d'hydrogène et d'oxyde de carbone assez faible étant comprise entre 1 et 40 bars environ et le rapport molaire $H_2/CO$ compris entre 1/1 et 20/1 environ.

Le procédé peut être mis en œuvre en faisant arriver dans une zone réactionnelle un courant d'alimentation composé d'un mélange d'hydrogène et d'oxyde de carbone, de la charge oléfinique à hydroformyler et du catalyseur neuf ou recyclé, dissous dans un solvant ou dans les produits lourds de la réaction.

Le produit de réaction peut être récupéré du milieu réactionnel par distillation d'un courant liquide soutiré en continu du réacteur, les produits lourds contenant le catalyseur étant éventuellement recyclés comme déjà mentionné. On peut encore récupérer les produits de réaction en les extrayant directement du milieu réactionnel par entraînement dans un courant gazeux sortant de la zone de réaction, qui peut être, par exemple, du gaz de synthèse en excès. Ce procédé présente l'avantage de laisser le système catalytique en place mais il ne s'applique commodément que pour l'hydroformylation d'oléfines légères telles que propène, butènes ou pentènes.

En général les complexes sont introduits dès le début pour obtenir un gain d'activité substantiel par rapport aux catalyseurs connus. Lorsqu'on effectue une réaction OXO à partir des complexes rhodium et organophosphorés décrits précédemment sans introduire dès le départ le cobalt et le diène, on note une activité catalytique plus faible et cette activité décroît dans le temps. Si on ajoute alors le cobalt et le diène, cela permet de retrouver une activité importante. Ainsi selon cette dernière mise en œuvre l'addition de cobalt et de diène en cours de réaction peut aussi être considérée comme une régénération du système rhodium + triorganophosphoré déjà connu.

Les exemples suivants, sans caractère limitatif, illustrent l'invention.

Exemple 1

D'une manière générale les essais 1 à 19 suivants sont effectués dans un autoclave en inox de 200 ml équipé d'un agitateur, d'une prise de température et d'une canalisation destinée à l'alimentation en gaz. On introduit 20 ml de n-butanal, 43 mg d'hydridocarbonyl tristriphénylphosphine rhodium (H Rh CO $[P0_3]_3$) et 1,33 g de triphénylphosphine $P0_3$. On ajoute ensuite le cobalt et le diène dans des proportions décrites dans le tableau 1. Après fermeture de l'autoclave et mise en route de l'agitation, on chauffe l'autoclave pour atteindre une température de 95°C; la pression relative est alors de 1,8 bar.

On établit dans l'autoclave les pressions partielles suivantes:

| | |
|---|---|
| Propène | 2 bars |
| Oxyde de carbone | 0,7 bar |
| Hydrogène | 5,5 bars |

La pression relative totale, qui atteint alors 10 bars, est maintenue à cette valeur par addition en continu d'un mélange de gaz $CO/H_2$/propène dans un rapport 1/1/1. Les productivités sont exprimées en g d'aldéhyde/heure × g de rhodium. Les productivités figurant dans le tableau 1 ont des valeurs qui font abstraction d'éventuelles périodes d'induction liées à la formation vraisemblable in situ de l'espèce catalytique active.

Les essais 1 à 4 sont donnés à titre comparatif. Les essais 1 et 2 illustrent l'effet néfaste des diènes sur les catalyseurs classiques. Ils montrent que l'addition d'un diène à un système catalytique constitué d'un complexe du rhodium et d'une phosphine ou constitué d'un complexe du cobalt abaisse considérablement et même annule l'acitivité du catalyseur.

Tableau 1

| Essais | Cobalt exprimé en rapport atomique Co/Rh | | Diène exprimé en g/litre | | Productivité exprimée en g/hxgRh |
|---|---|---|---|---|---|
| 1 | 275 ppm Rh sans Co | | Ethyl-2 héxèn-2 al | 164 | 86 |
| 2 | 700 ppm de Co sous forme de $Co_2(CO)_8$ sans Rh | | Ethyl-2 héxèn-2 al | 164 | 0 |
| 3 | 275 ppm Rh sans Co | | | | 531 |
| 4 | $Co_2(CO)_8$ | 5/1 | | | 616 |
| 5 | $Co_2(CO)_8$ | 5/1 | Ethyl-2 héxèn-2 al | 164 | 725 |
| 6 | $Co_2(CO)_8$ | 1/1 | Ethyl-2 héxèn-2 al | 164 | 775 |
| 7 | $Co_2(CO)_8$ | 5/1 | Acroleine | 0,4 | |
| 8 | $Co_2(CO)_8$ | 5/1 | Oxyde de Mesityle | 168 | 797 |
| 9 | $Co_2(CO)_8$ | 5/1 | Butadiène-1,3 | 7,8 | 651 |
| 10 | $Co_2(CO)_8$ | 5/1 | Diphényl-1,4 butadiène-1,3 | 1,5 | 711 |
| 11 | $Co_2(CO)_8$ | 5/1 | Diméthyl-1,5 2 pyrrole-carbonitrile | 9 | 636 |
| 12 | $Co_2(CO)_8$ | 5/1 | Hydroquinone | 1,3 | 674 |
| 13 | $Co_2(CO)_8$ | 5/1 | Cyclopentadiène | 44,4 | 623 |
| 14 | $(C_5H_5)$ Co $(CO)_2$ | 5/1 | | | 723 |
| 15 | (Ac. Ac.)$_2$ CO(II) | 5/1 | Ethyl-2 héxèn-2 al | 33 | 835 |
| 16 | $Co_2(CO)_8$ | 5/1 | Triphényl-1,2,5 phosphole | 2,2 | 652 |
| 17 | $Co_2(CO)_8$ | 5/1 | Pentephényl-1,2,3,4,5 phosphole | 3,3 | 780 |
| 18 | $Co_2(CO)_8$ | 5/1 | Benzoquinone | 0,7 | 841 |
| 19 | $Co_2(CO)_8$ | 5/1 | Furfural | 98,6 | 720 |

Exemple 2

Les essais 20 à 31 suivants sont effectués dans le même type d'appareillage permettant des essais longue durée et selon les mêmes conditions opératoires que les essais de l'exemple 1. Le rapport phosphore/rhodium est voisin de 100. Dans cette série d'essais on ajoute le système cobaltdiène après un certain temps de vieillissement du catalyseur ($HRhCO[P0_3]_3$).

Tableau 2

| Essais | Temps de vieillissement en heures | [RH] en ppm | Cobalt exprimé en rapport atomique Co/Rh | | Diène exprimé en g/litre | | Productivité en g/hxgRh |
|---|---|---|---|---|---|---|---|
| 20 | 0 | 440 | | | | | 358 |
| 21 | 394 | 565 | | | | | 241 |
| 22 | 394 | 452 | $Co_2(CO)_8$ | 5/1 | Ethyl-2 héxèn-2 al | 146 | 419 |
| 23 | 394 | 452 | $Co_2(CO)_8$ | 5/1 | Acétylacétone | 167 | 681 |
| 24 | 654 | 520 | | | | | 200 |
| 25 | 654 | 420 | $Co_2(CO)_8$ | 5/1 | Ethyl-2 héxèn-2 al | 146 | 427 |
| 26 | 654 | 420 | $Co_2(CO)_8$ | 5/1 | Ethyl-2 méthyl-4 pentèn-2 al | 146 | 369 |
| 27 | 654 | 420 | $Co_2(CO)_8$ | 5/1 | Oxyde de mésityle | 146 | 336 |
| 28 | 789 | 500 | | | | | 174 |
| 29 | 789 | 400 | $Co_2(CO)_8$ | 5/1 | Ethyl-2 héxèn-2 al | 146 | 318 |
| 30 | 789 | 400 | $Co_2(CO)_8$ | 1/1 | Ethyl-2 héxèn-2 al | 146 | 292 |
| 31 | 789 | 500 | $Co_2(CO)_8$ | 5/1 | Butadiène-1,3 | 16,8 | 269 |

Exemple 3

Les essais 32 à 34 suivants concernent l'hydroformylation d'héxène-1, et, ont été effectués dans un autoclave en inox de 200 ml équipé d'un agitateur, d'une prise de température et d'une canalisation destinée à l'alimentation en gaz. On introduit 10 ml de tolène, 10 ml d'héxène-1, 40 mg d'hydridocarbonyltristriphénylphosphine rhodium (H Rh $CO(P0_3)_3$) et 1,2 g de triphénylphosphine ($P0_3$). On ajoute ensuite le cobalt et le diène dans les proportions indiquées dans le tableau 3. Après fermeture et mise en route de l'agitation, on chauffe l'autoclave pour atteindre une température de 80°C; la pression relative est de 1 bar. On établit dans l'autoclave les pressions partielles suivantes:

– Monoxyde de carbone 1 bar
– Hydrogène 5 bars

La pression relative totale, qui atteint alors 7 bars, est maintenue à cette valeur par addition en continu d'un mélange de gaz $H_2/CO$ dans un rapport 1/1. Après 25 minutes de réaction, on coupe l'agitation, l'alimentation en gaz, on refroidit l'autoclave et on dégaze. Le mélange réactionnel est analysé par chromatographie en phase gazeuse et la productivité est exprimée en grammes d'aldéhydes formés par heure er par gramme de rhodium (g/hxgRh).

L'exemple 32 est donné à titre comparatif.

| Essais | Cobalt exprimé en rapport atomique Co/Rh | | Diène exprimé en g/litre | | Productivité exprimée en g/hxgRh |
|---|---|---|---|---|---|
| 32 | 275 ppm Rh sans Co | | | | 1007 |
| 33 | $C_5H_5\,Co(CO)_2$ | 10/1 | | | 1791 |
| 34 | $Co_2(CO)_8$ | 5/1 | pentaphényl-1,2,3,4,5 phosphole | 3 | 2456 |

**Revendications**

1. Système catalytique pour l'hydroformylation des oléfines constitué d'une combinaison complexe (1) de rhodium, d'oxyde de carbone et de ligand triorganophosphoré, d'un composé (2) de cobalt et d'un composé triorganophosphoré (3), caractérisé en ce qu'il contient au moins un composé (4) qui est un diène conjugué ou un composé générant in situ en cours de réaction d'hydroformylation un diène conjugué, ledit diène conjugué étant choisi dans les molécules aliphatiques, alicycliques, hétérocycliques et aromatiques possédant dans leur structure le motif

$-\underset{|}{C}=\underset{|}{C}-\underset{|}{C}=\underset{|}{C}-$ ou $-\underset{|}{C}=\underset{|}{C}-\underset{|}{C}=O$,

et en ce que le diène conjugué ne se trouve pas en combinaison complexe avec le rhodium ou le cobalt.

2. Procédé d'hydroformylation des oléfines à une température comprise entre 60 et 150°C sous une pression totale d'hydrogène et d'oxyde de carbone inférieure à 40 bars avec un rapport molaire $H_2/CO$ compris entre 1/1 et 20/1 caractérisé en ce qu'on utilise un système catalytique selon la revendication 1.

3. Procédé d'hydroformylation des oléfines à une température comprise entre 60 et 150°C sous

une pression totale d'hydrogène et d'oxyde de carbone inférieure à 40 bars avec un rapport molaire $H_2/CO$ compris entre 1/1 et 20/1 en présence comme catalyseur d'une combinaison complexe (1) de rhodium, d'oxyde de carbone et de ligand triorganophosphoré et d'un composé triorganophosphoré (3) caractérisé en ce qu'en cours d'hydroformylation on complète le catalyseur avec un composé (2) du cobalt et au moins un diène conjugué (4).

4. Procédé selon la revendication 3 caractérisé en ce qu'on complète le catalyseur avec un composé (2) du cobalt et un composé générant in situ un diène conjugué.

**Patentansprüche**

1. Katalysatorsystem für die Hydroformylierung von Olefinen, bestehend aus einer Komplexverbindung (1) von Rhodium, Kohlenoxid und einem Tri-Organophosphor-Liganden, einer Kobaltverbindung (2) und einer Tri-Organophosphor-Verbindung (3), dadurch gekennzeichnet, dass es mindestens eine Verbindung (4) enthält, die ein konjugiertes Dien oder eine im Laufe der Hydroformylierungsreaktion in situ ein konjugiertes Dien entwickelnde Verbindung ist, wobei das konjugierte Dien ausgewählt ist aus den aliphatischen, alicyklischen, heterocyklischen und aromatischen Molekülen, die in ihrer Struktur die Grundeinheit

$$-\underset{|}{C}=\underset{|}{C}-\underset{|}{C}=\underset{|}{C}- \quad \text{oder} \quad -\underset{|}{C}=\underset{|}{C}-\underset{|}{C}=O$$

besitzen, und dass das konjugierte Dien sich nicht in einer Komplexverbindung mit dem Rhodium oder dem Kobalt befindet.

2. Verfahren zur Hydroformylierung von Olefinen bei einer Temperatur zwischen 60 und 150°C, unter einem Wasserstoff/Kohlenoxid-Gesamtdruck unter 40 bar und bei einem Molverhältnis $H_2/CO$ zwischen 1/1 und 20/1, dadurch gekennzeichnet, daß man ein Katalysatorsystem nach Anspruch 1 verwendet.

3. Verfahren zur Hydroformylierung von Olefinen bei einer Temperatur zwischen 60 und 150°C, unter einem Wasserstoff/Kohlenoxid-Gesamtdruck unter 40 bar, bei einem Molverhältnis $H_2/CO$ zwischen 1/1 und 20/1 und in Gegenwart einer Komplexverbindung (1) von Rhodium, Kohlenoxid und einem Tri-Organophosphor-Liganden sowie einer Tri-Organophosphor-Verbindung (3) als Katalysator, dadurch gekennzeichnet, dass man im Laufe der Hydroformylierung den Katalysator mit einer Kobaltverbindung (2) und mindestens einem konjugierten Dien (4) ergänzt.

4. Verfahren nach Anspruch 3, dadurch gekennzeichnet, daß man den Katalysator mit einer Kobaltverbindung (2) und einer in situ ein konjugiertes Dien entwickelnden Verbindung ergänzt.

**Claims**

1. A catalytic system for the hydroformylation of olefins, comprising; (1) a complex compound of rhodium, carbon monoxide and triorganophosphorous ligand, (2) a compound of cobalt and (3) a triorganophosphorous compound, characterised in that it contains at least one compound (4) which is conjugated diene or a compound which generates a conjugated diene in situ during the course of a hydroformylation reaction, the said conjugated diene being chosen from aliphatic, alicyclic, heterocyclic and aromatic molecules which contain within their structure the group

$$-\underset{|}{C}=\underset{|}{C}-\underset{|}{C}=\underset{|}{C}- \quad \text{or} \quad -\underset{|}{C}=\underset{|}{C}-\underset{|}{C}=O$$

and in that the conjugated diene does not form part of a complex compound with rhodium or cobalt.

2. A method for hydroformylation of olefins at a temperature between 60 and 150°C under a total head of hydrogen and carbon monoxide of less than 40 bars with a molar ratio $H_2/CO$ of between 1/1 and 20/1, characterised in that a catalytic system according to claim 1 is used.

3. A method for hydroformylation of olefins at a temperature between 60 and 150°C under a total head of hydrogen and carbon monoxyde of less than 40 bars with a molar ratio $H_2/CO$ of between 1/1 and 20/1, in the presence of a catalyst consisting of a complex compound (1) of rhodium, carbon monoxyde and triorganophosphorous ligand and a triorganophosphorous compound (3), characterised in that during the course of hydroformylation the catalyst is supplemented with a compound (2) of cobalt and at least one conjugated diene (4).

4. A method according to claim 3, characterised in that the catalyst is supplemented with a compound (2) of cobalt and a compound which generates a conjugated diene in situ.